# EUROPEAN PATENT APPLICATION

(11) **EP 4 349 961 A1**
(43) Date of publication of application: **10.04.2024**
(21) Application number: 22816193.1
(22) Date of filing: 02.06.2022
(51) Int. Cl.: C12N 1/20, C05F 9/00, C05F 11/08

(54) **MICROBIAL PREPARATION PRODUCTION METHOD, MICROBIAL PREPARATION, AND COMPOST PRODUCTION METHOD**

(30) Priority: 02.06.2021 JP 2021093087; 02.06.2021 JP 2021093088
(71) Applicant: Taiyo Yuka Co., Ltd., Tokyo, 175-0091 (JP)
(72) Inventor: ISHIDA, Yohei, Tokyo 175-0091 (JP); GOTO, Tamaki, Tokyo 175-0091 (JP); FUJII, Katsuhiko, Tokyo 163-8677 (JP); OKURA, Toshinori, Tokyo 163-8677 (JP)
(74) Representative: Michalski Hüttermann & Partner Patentanwälte mbB
(86) International application number: PCT/JP2022/022475
(87) International publication number: WO 2022/255448

(57) **Abstract**

The present invention adopts a microbial preparation production method comprising: a step for mixing crushed waste gypsum with a culture medium containing microorganisms to obtain a mixed liquid; and a step for culturing the mixed liquid. The particle diameter of the crushed waste gypsum is at most 30 mm.

## Description

### BACKGROUND

### 1. TECHNICAL FIELD

The present invention relates to a production method of a microbial preparation, a microbial preparation, and a compost production method.

The present application claims priority based on Japanese Patent Application No. 2021-093087 and No. 2021-093088 filed in Japan on June 2, 2021, and the contents thereof are incorporation herein.

### 2. RELATED ART

Gypsum board is a material made of gypsum as a core material, covered on both sides with board paper, and molded into a plate-like shape. Gypsum board has excellent properties such as fire protection, fire resistance, and sound insulation, so it is widely used as a building material such as interior decoration material.

Approximately 1 million tons of gypsum board is discarded as waste gypsum annually, and there is a demand for reusing this. For example, Patent Document 1 describes a method of crushing gypsum board, separating and collecting pieces of paper, putting the pieces of paper in water, and producing paper material from recollected pulp. In addition, Patent Document 2 describes using an aqueous solution of gypsum as a fertilizer and supplying a calcium component to a plant.

### PRIOR ART DOCUMENTS

### PATENT DOCUMENTS

Patent Document 1: Japanese Patent No. 6664636
Patent Document 2: Japanese Patent No. 3016092

### SUMMARY CLAUSE

### OBJECT OF THE INVENTION

However, in the method of reusing gypsum board according to Patent Document 1, although the pulp included in the gypsum board can be reused, the gypsum (that is, calcium sulfate) must be discarded and cannot be reused.

In addition, the aqueous solution of gypsum according to Patent Document 2 does not include organic fertilizer. When the aqueous solution of gypsum according to Patent Document 2 is used with excessive addition of inorganic fertilizer such as nitrogen, phosphorus, potassium, soil damage may occur.

Thus, an object of the present invention is to make effective utilization of waste gypsum possible, and to provide a technique of producing a microbial preparation having organic matter decomposition ability and a technique of producing compost that can improve yield of agricultural crops.

### MEANS FOR SOLVING PROBLEMS

In order to achieve the above object, each aspect of the present invention employs the configuration below.

A production method of a microbial preparation according to the present invention includes mixing crushed waste gypsum with a culture medium including microorganisms to obtain a mixed liquid, and culturing the mixed liquid, and particle diameter of the crushed waste gypsum is 30 mm or less.

According to the production method of the microbial preparation, proliferation speed of the microorganisms can be improved due to the mixed liquid including crushed waste gypsum. In addition, according to the production method of the microbial preparation, a microbial preparation having sufficient decomposition ability of organic matter can be obtained at a low cost.

In addition, according to the production method of the microbial preparation, by adding waste gypsum to microorganisms and culturing it, enzyme activity in the microorganisms such as of endocellulase and hemicellulase that decompose refractory organic matter is increased.

In addition, by adding this microbial preparation to cultivation soil of a crop, refractory organic matter in the soil is decomposed. As a result, it is possible to improve crop growth.

In the production method of the microbial preparation, it is preferable to culture the mixed liquid under aerobic conditions.

By culturing under aerobic conditions, proliferation of sulfate reducing bacteria can be suppressed in the cultured mixed liquid. As a result, during culturing the mixed liquid, generation of hydrogen sulfide can be suppressed. In addition, when the obtained microbial preparation is used, a risk of generation of hydrogen sulfide can be reduced.

The mixed liquid is preferably cultured at a temperature of 10 to 50 degrees Celsius. By culturing at a temperature of 10 to 50 degrees Celsius, proliferation speed of the microorganisms can be increased, and microbiota included in the obtained microbial preparation becomes suitable for organic matter decomposition.

The culture medium preferably includes one type or more selected from a group formed of starch and plant fiber, and calcium sulfate.

In this way, proliferation speed of the microorganisms can be increased, and microbiota included in the obtained microbial preparation becomes suitable for organic matter decomposition.

The production method of the microbial preparation further preferably includes determining that arsenic and cadmium content in the waste gypsum is a certain value or less.

Due to the arsenic and cadmium content in the waste gypsum being the upper limit value or less, the microbial preparation obtained by the production method of the microbial preparation is harmless to the human body and can be used safely.

Content of the waste gypsum in relation to total mass of the mixed liquid is preferably 0.01 to 30% by mass. In this way, proliferation speed of the microorganisms is improved and the microbial preparation can be produced in less time.

In the production method of the microbial preparation, microorganisms collected from activated sludge are preferably used.

By using microorganisms collected from activated sludge, organic matter decomposition ability of the microbial preparation can be made sufficient, and enzyme activity on refractory organic matter can be increased.

A microbial preparation according to the present invention includes microorganisms, crushed waste gypsum, and water, and particle diameter of the crushed waste gypsum is 30 mm or less.

The microbial preparation of the present embodiment can exhibit sufficient decomposition ability of organic matter.

A compost production method according to the present invention is a compost production method including adding microorganisms and waste gypsum to organic matter and fermenting it.

According to the compost production method, by using the above described microorganisms and waste gypsum, organic matter can be sufficiently decomposed.

In addition, according to the compost production method, by adding microorganisms and waste gypsum to organic matter and fermenting it, decomposition of refractory organic matter in the organic matter is enhanced. In this way, effect of the compost is increased, and it is possible to improve crop growth.

In addition, since the compost obtained by the compost production method includes a calcium component and a sulfur component supplied from the waste gypsum in addition to the decomposed organic matter, harvest amount of agricultural crops and flavor of agricultural crops can be improved.

A composting material according to the present invention includes microorganisms, waste gypsum, and organic matter.

By fermenting the composting material according to the present invention, a compost that can improve harvest amount of agricultural crops and flavor of the harvest can be obtained.

A compost fermentation product according to the present invention is made by fermenting microorganisms, waste gypsum, and organic matter.

The compost fermentation product according to the present invention can improve harvest amount of agricultural crops and flavor of the harvest.

### EFFECT OF THE INVENTION

According to the present invention, waste gypsum can be effectively utilized, and a technique for producing a microbial preparation having organic matter decomposition ability and a technique for producing compost that can improve yield of agricultural crops can be provided.

### BRIEF DESCRIPTION OF THE DRAWINGS

FIG. 1 exemplifies an embodiment of a production method of a microbial preparation.
FIG. 2 exemplifies an embodiment of a production method of a microbial preparation.
FIG. 3 shows a graph of microbial species included in organic sludge and their OTU values.
FIG. 4 is a graph showing culturing results of microbiota collected from organic sludge.
FIG. 5 is a graph showing a result of ammonia decomposition using the microbial preparation of the present invention.
FIG. 6 shows a flow of sludge treatment.
FIG. 7 is a graph showing results of inputting the microbial preparation of the present invention in a sludge treatment tank and suppressing generation of hydrogen sulfide.
FIG. 8 shows results of measuring activity of enzymes that decompose refractory organic matter, regarding the microbial preparation of the present invention.

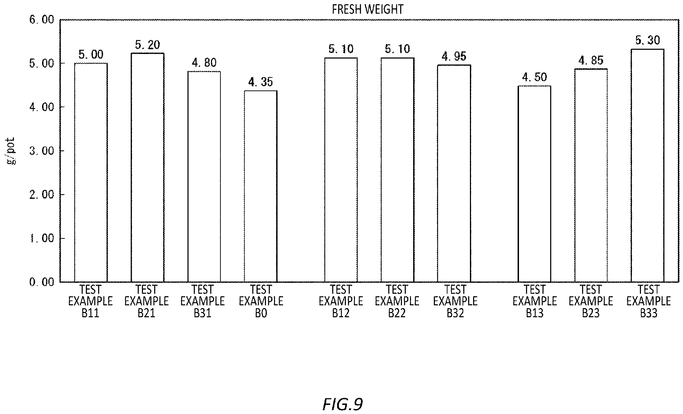

FIG. 9 is a graph showing fresh weight of crops cultivated using the microbial preparation of the present invention.
FIG. 10 is a graph showing fresh weight of crops cultivated using compost of the present invention.
FIG. 11 is a graph showing total nitrogen amount in soil and nitrate nitrogen amount in crops after cultivation, when the crops are cultivated using the compost of the present invention.
FIG. 12 is a graph showing electrical conductivity (EC) of soil after cultivation, when crops are cultivated using the compost of the present invention.

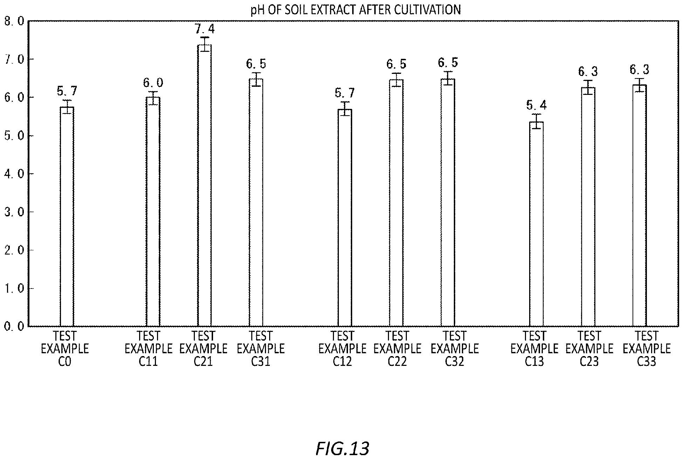

FIG. 13 is a graph showing pH of soil after cultivation, when crops are cultivated using the compost of the present invention.

### DETAILED DESCRIPTION OF EMBODIMENTS OF THE INVENTION

### (Production method)

In one embodiment, the present invention provides a production method of a microbial preparation including a process A of mixing a culture medium including microorganisms with crushed waste gypsum to obtain a mixed liquid, and a process B of culturing the mixed liquid, and particle diameter of the crushed waste gypsum is 30 mm or less.

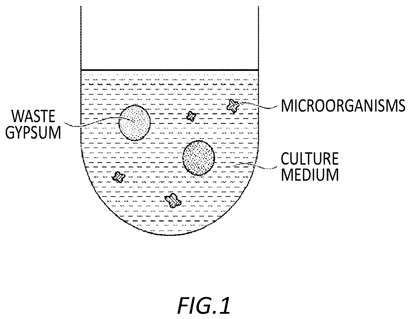

FIG. 1 illustrates an example of a production method of a microbial preparation according to the present embodiment. As shown in FIG. 1, by culturing microorganisms in a mixed liquid including waste gypsum and a culture medium, a microbial preparation is produced.

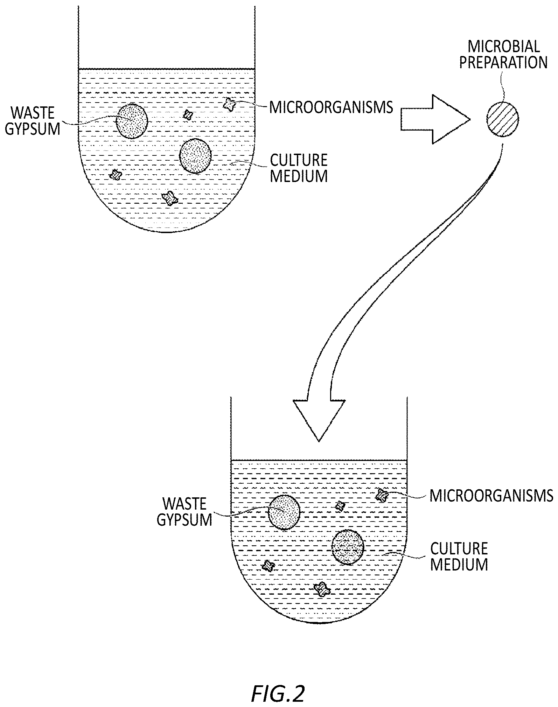

As exemplified in FIG. 2, the obtained microbial preparation may be subcultured to produce a microbial preparation.

### (Process A)

In process A, a mixed liquid is obtained by mixing crushed waste gypsum in a culture medium including microorganisms.

### (Microorganisms)

For the microorganisms, for example, thermophilic bacteria, actinomycetes, sulfur oxidizing bacteria, photosynthetic bacteria, nitrogen fixing bacteria and the like can be mentioned.

More specifically, for the microorganisms, genus Caldilinea, Candidate division OP10, genus Dechloromonas, genus Parvibaculum, genus Clostridium, genus Methanosphaera, genus Novosphingobium, genus Sporosarcina, genus Rhodococcus, genus Phaeospirillum, genus Treponema, genus Methanobacterium, genus Azospira, genus Methanosaeta, genus Meiothermus, genus Methylosinus, genus Longilinea, genus Rhodoplanes and the like can be mentioned.

In the present specification, microorganisms may be described as microbiota.

The microorganisms included in the culture medium of process A are preferably microorganisms collected from activated sludge of a sewage sludge treatment tank. For activated sludge, for example, those derived from excrement of animals including humans and sewage and sludge generated in grease traps generated in kitchens and the like can be mentioned.

The microorganisms may be a mutant of a microorganism isolated from nature, or may be a mutant of the above described microorganisms. Here, a "mutant" refers to a microorganism in which a mutation has been produced, spontaneously or artificially, in genes of an original microorganism. An artificial method for producing a genetic mutation is not particularly limited, and ultraviolet irradiation, radiation irradiation, chemical treatment with nitrous acid and the like, genetic engineering methods such as gene introduction and genome editing and the like can be exemplified.

For a suitable example of a mutant, a mutant in which a silent mutation is produced, a mutant in which a foreign gene has been introduced and the like can be mentioned. Genome sequence identity (homology) between the mutant and the original microorganism is preferably 97% or more, more preferably 98% or more, further preferably 99% or more, and further preferably 99.5% or more. 99.9% or more is particularly preferable.

The microorganisms included in the culture medium of process A may be one type or may be two types or more.

The culture medium of process A may include microorganisms other than the above described microorganisms (other microorganisms).

For example, for the other microorganisms, Bacillus subtilis, lactic acid bacteria, yeast, archaea and the like can be mentioned.

### (Waste gypsum)

Gypsum is a mineral having calcium sulfate as its principal component.

Gypsum is used as building material such as gypsum board. In the present specification, waste gypsum refers to waste material having gypsum as its principal component.

The waste gypsum included in the culture medium of process A may be crushed gypsum board and the like.

Particle diameter of the waste gypsum is preferably 30 mm or less, more preferably 20 mm or less, further preferably 10 mm or less, particularly preferably 3 mm or less, and most preferably 1 mm or less. Note that particle diameter being A mm or less in the present specification is defined as including only less than 0.1% by mass of gypsum particles with a length exceeding A mm in the waste gypsum.

Due to particle diameter of the waste gypsum being the upper limit value or less, area of the waste gypsum that comes in contact with the culture medium increases. In this way, proliferation speed of the microorganisms can be improved.

Although a lower limit value of particle diameter of the waste gypsum is not particularly limited, for example, it may be 10 µm.

To crush the gypsum board, a commercially available gypsum board crusher may be used, or another crushing means may be used. Crushed waste gypsum is used as raw material without removing board paper, after removing masses with large particle diameter with a sieve having a predetermined opening diameter if necessary. Since there is a possibility that crushed waste gypsum includes a foreign body such as screws, nails, staples, metal plates, stud materials, and mortar, and these foreign bodies are preferably removed.

In this way, the microbial preparation obtained by the production method of the present embodiment can be used more safely. However, if the above mentioned foreign body is in a small amount, its presence has no effect on proliferation of the microorganisms.

Generally, waste gypsum obtained by crushing gypsum board and the like includes board paper, glue and the like. The waste gypsum included in the culture medium of process A preferably includes board paper and glue.

By including board paper and glue, a nutrient source for the microorganisms is supplied in the mixed liquid. As a result, proliferation speed of microorganisms in process B is improved.

The waste gypsum included in the microbial preparation of the present embodiment may be one type or more selected from a group formed of gypsum dihydrate (CaSO₄ • 2H₂O), gypsum hemihydrate (CaSO₄ • 1/2H₂O), and gypsum anhydrite (CaSO₄), is preferably one type or more selected from a group formed of gypsum dihydrate and gypsum anhydrite, and is further preferably gypsum dihydrate.

Gypsum used for building material such as gypsum board is gypsum dihydrate. Firing treatment may be performed on the crushed waste gypsum. By firing gypsum dihydrate obtained by crushing gypsum board and the like, gypsum hemihydrate and gypsum anhydrite are obtained. Firing method may be a method well known by one skilled in the art.

Since gypsum dihydrate and gypsum anhydrite do not harden when mixed with water, the microbial preparation can be produced more easily.

### (Culture medium)

The culture medium of process A is not particularly limited as long as microorganisms can proliferate, and one well known by one skilled in the art can be used.

The culture medium may include a nutrient source such as a carbon source, nitrogen source, inorganic salt, and humic substance. The nutrient source may be supplied from waste gypsum, organic sludge or activated sludge.

For example, for the carbon source, glucose, fructose, sucrose, maltose, lactose, dextran, starch, pyruvate, acetate, plant fiber and the like can be mentioned. For the plant fiber, cellulose, lignin, hemicellulose and the like can be mentioned.

The starch may be supplied from glue included in waste gypsum. The plant fiber may be supplied from board paper included in waste gypsum.

For the nitrogen source, ammonium sulfate, ammonium chloride, ammonium salt such as ammonium nitrate, nitrates, amino acids, peptone, casein, casamino acids, urea, yeast extract, corn steep liquor, wheat germ, sugar cane pulp, beer lees, cottonseed meal, wheat bran, soybean flour, rice bran, broth, meat extract, fishmeal and the like can be mentioned.

For the inorganic salt, sodium phosphate, sodium chloride, sodium salt such as molybdenum sodium, potassium salt such as potassium phosphate and potassium chloride, calcium salt such as calcium chloride, calcium phosphate, and calcium sulfate, magnesium salt such as magnesium chloride and magnesium sulfate, a transition metal salt such as iron chloride and the like can be mentioned.

The culture medium preferably includes one type or more selected from a group formed of starch and plant fiber as the carbon source.

The culture medium preferably includes calcium sulfate as the inorganic salt.

Process A may be a process in which a mixed liquid is obtained by mixing activated sludge with crushed waste gypsum. That is, the culture medium including the microorganisms may be activated sludge.

For the culture medium including the microorganisms, when activated sludge is used, although content of the activated sludge (% by mass) is not particularly limited as long as an effect of the present invention is achieved, it may be 10 to 95% by mass, may be 30 to 95% by mass, or may be 50 to 95% by mass, in relation to total mass of the mixed liquid (100% by mass).

In the mixed liquid of process A, content of the waste gypsum in relation to the total mass of the mixed liquid is preferably 0.01% to 30% by mass, more preferably 0.1% to 20% by mass, and further preferably 0.1% to 10% by mass.

Due to the content of the waste gypsum being within the above described range, proliferation speed of the microorganisms can be improved.

### (Process B)

In process B, the mixed liquid obtained in process A is cultured.

Culture temperature of process B is preferably 10 to 50 degrees Celsius, more preferably 20 to 45 degrees Celsius, further preferably 25 to 40 degrees Celsius, particularly preferably 28 to 35 degrees Celsius, and most preferably 28 to 30 degrees Celsius.

Due to the culture temperature being within the range, proliferation speed of the microorganisms is improved and the microbial preparation can be produced in less time.

Process B may be performed under anaerobic conditions or may be performed under aerobic conditions.

Process B is preferably performed under aerobic conditions.

By culturing under aerobic conditions, proliferation of sulfate reducing bacteria can be suppressed in the cultured mixed liquid. As a result, in process B, generation of hydrogen sulfide can be suppressed. In addition, when the obtained microbial preparation is used, a risk of generation of hydrogen sulfide can be reduced.

In process B, the mixed liquid may be cultured while being shaken, the mixed liquid may be cultured with aeration, the mixed liquid may be cultured while being stirred using a stirrer and the like, or may be cultured standing still.

In process B, pH of the mixed liquid is preferably 4 to 10, and more preferably 6 to 9. In this way, proliferation of sulfate reducing bacteria can be suppressed in the cultured mixed liquid. As a result, in process B, generation of hydrogen sulfide can be suppressed. In addition, when the obtained microbial preparation is used, a risk of generation of hydrogen sulfide can be reduced.

The production method of the microbial preparation of the present embodiment, upstream to process A, may include a process for determining that arsenic and cadmium content in the waste gypsum is a certain value or less.

Measurement of arsenic and cadmium content in waste gypsum may be a method well known by one skilled in the art, and for example, may be fluorescent X-ray spectroscopy.

Arsenic content in the waste gypsum included in the microbial preparation of the present embodiment is preferably 50 ppm or less, more preferably 10 ppm or less, further preferably 5 ppm or less, and particularly preferably 2 ppm or less.

Cadmium content in the waste gypsum included in the microbial preparation of the present embodiment is preferably 10 ppm or less, more preferably 3 ppm or less, and further preferably 1 ppm or less.

Due to the arsenic and cadmium content in the waste gypsum being the upper limit value or less, the microbial preparation obtained by the production method of the present embodiment is harmless to the human body and can be used safely.

According to the production method of the microbial preparation of the present embodiment, proliferation speed of the microorganisms can be improved due to the mixed liquid including crushed waste gypsum.

In addition, according to the production method of the microbial preparation of the present embodiment, a microbial preparation having sufficient decomposition ability of organic matter can be obtained at a low cost. This microbial preparation can be suitably used for decomposition of organic matter such as ammonia, reducing generation of hydrogen sulfide in a sludge treatment tank and the like.

According to the production method of the microbial preparation of the present embodiment, microorganisms are cultured in a culture medium including crushed waste gypsum. The waste gypsum includes calcium sulfate and cardboard, and the cardboard includes starch and dietary fiber. According to this production method, since dietary fiber derived from the waste gypsum is included in a mixed solution, enzyme activity in microorganisms on refractory organic matter is increased.

Although the reason for such an effect is not clear, it is presumed as follows.

Many types of microorganisms exist in microbiota. By adding waste gypsum to microbiota and culturing it, proportion of microbial species that can decompose refractory organic matter and proliferate increases. In addition, expression level and activity of degrading enzymes for refractory organic matter in the microorganisms increases. It is presumed that as a result, the culture solution that has been cultured with waste gypsum added to the microbiota has expression levels and activity of degrading enzymes for refractory organic matter that are higher than a culture solution that has been cultured with calcium sulfate and industrial glue added to the microbiota.

### (Microbial preparation)

In one embodiment, the present invention provides a microbial preparation including microorganisms, crushed waste gypsum, and water, and particle diameter of the crushed waste gypsum is 30 mm or less.

The microbial preparation of the present embodiment may be obtained by the (Production method) described above.

### (Microorganisms)

For microorganisms included in the microbial preparation of the present embodiment, those described above in the (Production method) can be mentioned.

In the microbial preparation of the present embodiment, although microorganism content is not particularly limited as long as it is included to an extent that the microbial preparation achieves an effect of the invention of the present application, it may be 0.0001 to 1% by mass in relation to total mass of the microbial preparation.

### (Waste gypsum)

For the waste gypsum included in the microbial preparation of the present embodiment, that described above in the (Production method) can be mentioned.

In the microbial preparation of the present embodiment, although waste gypsum content is not particularly limited as long as it is included to an extent that the microbial preparation achieves an effect of the invention of the present application, it is preferably 0.01% to 30% by mass, more preferably 0.1% to 10% by mass, and further preferably 0.3% to 10% by mass in relation to total mass of the microbial preparation.

The microbial preparation of the present embodiment may include a culture medium. For the culture medium, those described above in the (Production method) can be mentioned.

Activated sludge includes microorganisms and water.

The microbial preparation of the present embodiment may be a preparation including activated sludge and crushed waste gypsum, and particle diameter of the crushed waste gypsum is 30 mm or less.

### (Other components)

The microbial preparation of the present embodiment may include a carbon source, nitrogen source, inorganic salt and the like.

For example, for the carbon source, glucose, fructose, sucrose, maltose, lactose, dextran, starch, pyruvate, acetate, plant fiber and the like can be mentioned.

For the nitrogen source, ammonium sulfate, ammonium chloride, ammonium salt such as ammonium nitrate, nitrates, amino acids, peptone, casein, casamino acids, urea, yeast extract, corn steep liquor, wheat germ, sugar cane pulp, beer lees, cottonseed meal, wheat bran, soybean flour, rice bran, broth, meat extract, fishmeal and the like can be mentioned.

For the inorganic salt, sodium phosphate, sodium chloride, sodium salt such as molybdenum sodium, potassium salt such as potassium phosphate and potassium chloride, calcium salt such as calcium chloride, calcium phosphate, and calcium sulfate, magnesium salt such as magnesium chloride and magnesium sulfate, a transition metal salt such as iron chloride and the like can be mentioned.

The microbial preparation of the present embodiment may include an arbitrary component similarly to a typical microbial preparation and be formulated into a form such as a powder, wettable powder, granule, emulsion, solution, suspension, flowable, and coating.

The microbial preparation of the present embodiment may include a carrier.

For example, for the carrier, a carbine, mineral, metal or metal salt, silicon, polymer and the like can be mentioned. More specifically, charcoal, sand, diatomaceous earth, zeolite, perlite, bentonite, ceramics, alumina, gypsum, silica gel, glass and the like can be mentioned.

The microbial preparation of the present embodiment may be the above described microorganisms cultured in advance to which the above described culture medium, arbitrary component, and waste gypsum are added.

The microorganisms included in the microbial preparation of the present embodiment may be subcultured.

The method of subculture may be a method of culture using the above described culture medium, or may be a method of culture using a culture medium including waste gypsum.

The microbial preparation of the present embodiment can be used for decomposition of organic matter.

Alternatively, the microbial preparation of the present embodiment may be used for decomposing waste including organic matter.

For example, for waste including organic matter, waste water, food waste and the like can be mentioned.

For example, for waste water, domestic waste water, waste water generated in livestock housing and the like, industrial waste water generated in food factories, drinking water factories and the like, waste water in sewage treatment plants and the like can be mentioned.

More specifically, for organic matter, sugar, fat, protein and the like can be mentioned.

The microbial preparation of the present embodiment can exhibit sufficient decomposition ability of organic matter.

The microbial preparation of the present embodiment can be suitably used for decomposition of organic matter such as ammonia, reducing generation of hydrogen sulfide in a sludge treatment tank and the like.

In the microbial preparation of the present embodiment, by adding waste gypsum to microbiota and culturing it, enzyme activity in the microorganisms such as of endocellulase and hemicellulase that decompose refractory organic matter is increased.

For example, the microbial preparation of the present embodiment can be used by being added to cultivation soil of a crop as liquid fertilizer. In this case, the liquid fertilizer may be supernatant of the microbial preparation of the present embodiment.

By adding the microbial preparation of the present embodiment to cultivation soil of a crop, it is possible to improve crop growth. The reason for such an effect is presumed as follows.

Typically, in soil, easily decomposable organic matter is in a state of decomposition by microorganisms existing in the soil. By adding a microbial preparation produced by culturing microbiota with waste gypsum to soil, refractory organic matter in the soil is decomposed.

This microbial preparation includes a calcium component and a sulfur component supplied from waste gypsum. In addition, calcium sulfate included in waste gypsum shows neutrality.

It is presumed by this synergistic effect, crop growth is improved in a soil to which the microbial preparation is added.

### (Compost production method)

In one embodiment, the present invention provides a compost production method including a process for adding microorganisms and waste gypsum to organic matter and fermenting it. The compost production method according to the present embodiment may be a compost production method including a process for adding supernatant of a mixed liquid of microorganisms and waste gypsum to organic matter and fermenting it.

In the present specification, "compost" refers to an organic material obtained by decomposing and fermenting organic matter such as livestock excrement, raw refuse, and food residue.

The compost production method according to the present embodiment obtains compost by fermenting compost raw material.

In the compost production method according to the present embodiment, organic matter, microorganisms, and waste gypsum are used as compost raw material.

As long as an effect of the present invention is achieved, the order in which the microorganisms and the waste gypsum are added to the organic matter is not particularly limited. After a mixture of waste gypsum added to organic matter is obtained, microorganisms may be added to the mixture. Alternatively, after a mixture of microorganisms added to organic matter is obtained, waste gypsum may be added to the mixture.

It is preferable that after the mixture of waste gypsum added to organic matter is obtained, the microorganisms are added to the mixture. In this way, proliferation of microorganisms included in microorganisms can be improved. In addition, time until the compost is obtained is reduced, and compost of good quality can be obtained.

Alternatively, the compost production method according to the present embodiment may include a process for adding the above described microbial preparation to organic matter and fermenting it. The compost production method according to the present embodiment may include a process for adding supernatant of the above described microbial preparation to organic matter and fermenting it.

For microorganisms used in the compost production method according to the present embodiment, those microorganisms described above in the (Microbial preparation) can be mentioned.

The microorganisms are preferably microorganisms collected from activated sludge of a sewage sludge treatment tank.

Although content of microorganisms is not particularly limited as long as an effect of the present invention is achieved, for example, it may be 0.001 to 10% by mass in relation to total amount of compost raw material (100% by mass).

The compost production method according to the present embodiment may be a compost production method including a process for adding activated sludge and waste gypsum to organic matter and fermenting it.

In this case, although content of activated sludge is not particularly limited as long as an effect of the present invention is achieved, for example, it may be 1 to 50% by mass, may be 5 to 40% by mass, or may be 5 to 30% by mass in relation to total amount of compost raw material (100% by mass).

Alternatively, the compost production method according to the present embodiment may be a compost production method including a process for adding supernatant of a mixed liquid of activated sludge and waste gypsum to organic matter and fermenting it.

For waste gypsum used in the compost production method according to the present embodiment, the waste gypsum described above in the (Microbial preparation) can be mentioned.

Although content of waste gypsum is not particularly limited as long as an effect of the present invention is achieved, for example, it may be 0.01 to 10% by mass, may be 0.1 to 10% by mass, or may be 0.5 to 5% by mass in relation to total amount of compost raw material (100% by mass).

### (Organic matter)

Organic matter used in the compost production method according to the present embodiment is not particularly limited as long as it can be fermented and composted.

For example, for organic matter, livestock excrement, food residue, wood residue, fishing waste and the like can be mentioned.

For example, for livestock excrement, cow manure, pig manure, chicken manure, horse manure and the like can be mentioned.

For example, for food residue, leftover food, raw refuse, vegetable scraps, beer lees, soybean curd refuse and the like can be mentioned.

For example, for fishing waste, fish bones, fish scraps, shellfish scraps, seaweed residue and the like can be mentioned.

For organic matter used in the compost production method according to the present embodiment, one type may be used, or two types or more may be used.

Although amount of water included in organic matter is not particularly limited as long as an effect of the present invention is achieved, it may be 10 to 90% by mass or may be 20 to 60% by mass in relation to total amount of organic matter (100% by mass).

Although content of organic matter is not particularly limited as long as effect of the present invention is achieved, it may be 60 to 99% by mass or may be 70 to 95% by mass in relation to total amount of compost raw material (100% by mass).

The compost production method according to the present embodiment may use a secondary material in addition to the above described microorganisms, waste gypsum, and organic matter as raw material of compost.

For example, for the secondary material, inorganic material such as zeolite, vermiculite, and perlite, and soil improvement material such as wood-based raw material such as rice husk and sawdust can be mentioned.

The compost production method according to the present embodiment, upstream to the process for adding microorganisms and waste gypsum to organic matter and fermenting it, may include a process for determining that arsenic and cadmium content in the waste gypsum is a certain value or less.

Measurement of arsenic and cadmium content in waste gypsum may be a method well known by one skilled in the art, and for example, may be fluorescent X-ray spectroscopy.

In the compost production method according to the present embodiment, arsenic content in the waste gypsum is preferably 50 ppm or less, more preferably 10 ppm or less, further preferably 5 ppm or less, and particularly preferably 2 ppm or less.

In the compost production method according to the present embodiment, cadmium content in the waste gypsum is preferably 10 ppm or less, more preferably 3 ppm or less, and further preferably 1 ppm or less.

Due to arsenic and cadmium content in the waste gypsum being the upper limit value or less, when a food crop is cultivated using compost obtained by the production method according to the present embodiment, arsenic concentration and cadmium concentration in the obtained food can be reduced. In this way, food obtained by cultivation using compost obtained by the production method according to the present embodiment can be ingested safely.

For example, the compost production method according to the present embodiment may include a pre-treatment process and a fermentation process as described below.

In the pre-treatment process, air permeability, moisture content, pH and the like of compost raw material may be adjusted. In addition, in the pre-treatment process, substances unsuitable for fermentation such as food packaging plastic and metal may be removed. In the fermentation process, organic matter of compost raw material is decomposed.

When animal excrement and the like having high moisture content is used as organic matter, moisture adjustment may be performed. By maintaining an appropriate amount of moisture content included in compost raw material, anaerobic fermentation can be suppressed, and generation of hydrogen sulfide and the like and generation of foul smells associated with anaerobic fermentation can be suppressed.

Although water content (% by mass) in compost raw material is not particularly limited as long as an effect of the present invention is achieved, for example, it may be 20 to 90%, may be 30 to 80%, or may be 40 to 70% in relation to total mass of compost raw material (100% by mass).

Particle diameter of compost raw material is not particularly limited as long as good quality compost can be produced, and for example, by setting particle diameter of the compost raw material referring to a technique well known to one skilled in the art, air permeability is improved and aerobic fermentation can be enhanced.

Generally in a fermentation process, by performing fermentation in which air permeability, moisture content, pH and the like are within an appropriate range, aerobic fermentation is performed. In an initial fermentation process (before switching), aerobic fermentation may proceed appropriately and temperature of compost raw material may be maintained at 60 degrees Celsius or more for a fixed period of time. In this way, pathogenic bacteria, parasite eggs and the like are killed, and weed seeds and the like that may be included in compost raw material can be inactivated.

In the fermentation process, although temperature of outside and inside where the compost raw material is disposed is not particularly limited as long as an effect of the present invention is achieved, for example, it may be 5 to 50 degrees Celsius, may be 10 to 45 degrees Celsius, or may be 20 to 45 degrees Celsius.

Although duration of the fermentation process is not particularly limited as long as an effect of the present invention is achieved, it may be 2 weeks to 2 years, or may be 3 weeks to 1 year.

For example, for equipment used in the fermentation process, a pile type, silo type, tunnel type, batch type and the like can be mentioned. In the fermentation process, compost raw material is preferably mixed in order to promote aerobic fermentation. For example, a method of mixing may be a method by cutback or may be a method using a mixer.

For example, in the fermentation process, for a method of permeating air to compost raw material, an air supply method, air intake method and the like can be mentioned.

For reducing time for compost raw material to ferment and organic matter to be decomposed, for example, when outside temperature is low, the compost raw material may be heated and warmed.

The compost production method according to the present embodiment can sufficiently decompose organic matter by using the above described microorganisms and waste gypsum.

As described above in the (Microbial preparation), by adding waste gypsum to microbiota and culturing it, enzyme activity in microorganisms that decompose refractory organic matter is increased.

According to the compost production method according to the present embodiment, by adding microorganisms and waste gypsum to organic matter and fermenting it, decomposition of refractory organic matter in organic matter is enhanced. It is presumed that in this way, effect of compost is increased, and crop growth is improved.

Since compost obtained by the compost production method according to the present embodiment includes a calcium component and a sulfur component supplied from the waste gypsum in addition to the decomposed organic matter, harvest amount of agricultural crops and flavor of agricultural crops can be improved.

Since waste gypsum has higher solubility in water than calcium carbonate used in general fertilizer, a calcium component of compost obtained by the compost production method according to the present embodiment is easily absorbed by agricultural crops.

In addition, while slaked lime used in general fertilizer dissolves in water and shows alkalinity, since calcium sulfate dissolved from waste gypsum shows neutrality, alkalinization of soil can be suppressed.

In the above described compost, since organic matter in the compost raw material is sufficiently fermented and has high ripeness, it is possible to increase germination rate.

In addition, by cultivating a crop using the above described compost, nitrogen compounds are more easily held in the cultivation soil. In this way, it is possible to increase nitrogen fixation of the crop.

It is presumed that by the above described synergistic effect, compost obtained by the compost production method according to the present embodiment improves crop growth.

### (Composting material)

In one embodiment, the present invention provides a composting material including microorganisms, waste gypsum, and organic matter.

For the microorganisms, waste gypsum, and organic matter included in the composting material according to the present embodiment, those described above in the (Compost production method) can be mentioned.

The composting material can also be the above described compost raw material.

A mixing ratio of the microorganisms, waste gypsum, and organic matter in the composting material may be a mixing ratio of microorganisms, waste gypsum, and organic matter in compost raw material.

By fermenting the composting material according to the present embodiment, a compost that can improve harvest amount of agricultural crops and flavor of the harvest can be obtained.

### (Compost fermentation product)

In one embodiment, the present invention provides a compost fermentation product made by fermenting microorganisms, waste gypsum, and organic matter.

The compost fermentation product according to the present embodiment may be obtained by fermenting the above described compost raw material.

The compost fermentation product according to the present embodiment can also be compost.

The compost fermentation product according to the present embodiment can improve harvest amount of agricultural crops and flavor of the harvest.

### (Culture soil)

In one embodiment, the present invention provides a culture soil including the above described compost fermentation product.

"Culture soil" refers to soil mixed with potting soil and fertilizer, and refers to a material for growing a plant instead of soil.

By cultivating agricultural crops using the culture soil according to the present embodiment, harvest amount of the agricultural crops and flavor of the harvest can be improved. Embodiment

The present invention will be described by an embodiment below, but the present invention is not limited to the embodiment below.

### (Materials and method)

### (Waste gypsum)

The waste gypsum used was one including gypsum and cardboard derived from gypsum board. The gypsum was crushed in a mortar, and the cardboard was cut to 10 x 1 mm. Particle diameter of the crushed gypsum was 1 mm or less.

### (Collection of liquid including microbiota)

Organic sludge collected from a waste water tank (building pit) was processed by an activated sludge method to obtain activated sludge. A 1000 mL liquid including the activated sludge was put in a sterilized 1000 mL wide-mouth plastic container using a dipper disinfected with ethanol. This wide-mouth plastic container was stored at 5 degrees Celsius. The collected activated sludge was considered to be a "liquid including microbiota".

### (Metagenomic analysis)

Metagenomic DNA was extracted from the microbiota, its base sequence was analyzed by a next-generation sequencer, and the microorganisms were phylogenetically classified. A composition ratio of the classified microorganisms was evaluated by calculating operational taxonomic unit (OTU).

### (Culture of microorganisms in advance)

20 µL of the stored liquid was inoculated to 20 mL of culture medium (Beef extract 10 g/L, Peptone 10 g/L, NaCl 5 g/L). This culture medium was cultured at 30 degrees Celsius for 48 hours.

### (Bacteria count test)

A normal broth agar culture medium (DIW 1000 mL, Beef extract 10 g, Peptone 10 g, NaCl 5 g, Bacto agar 20 g) was prepared.

20 µL of each culture solution obtained in the main culture was each inoculated to the normal broth culture medium with a cell spreader, and was cultured at 30 degrees Celsius for 24 hours. Then, colonies formed on the agar culture medium were counted. This process was repeated three times, and an average value of the number of bacteria was calculated as colony forming units (CFU).

### (Production of a microbial preparation)

First, a septic tank connected with four tanks (1 m³ capacity each) was prepared, and a last tank among the four tanks was considered a storage tank of a microbial preparation. An adsorbent and a stirring blower pump were disposed in each tank. The microbial preparation obtained by culturing was stored in the storage tank in an overflow manner.

Organic sludge collected from a building pit was used as sludge. 60 L of organic sludge was supplied to the septic tank per day, and aeration was performed with the blower pump during culturing of the microorganisms to adjust dissolved oxygen in the culture solution to more than zero.

Note that the liquid including microbiota collected from the storage tank was mixed with waste gypsum 10:1, and subcultured at a water temperature of 30 degrees Celsius for 24 hours. 1 L of the obtained culture solution was supplied to each of the four septic tanks once every 40 days.

### (Experimental example 1)

### (Metagenomic analysis of microbiota)

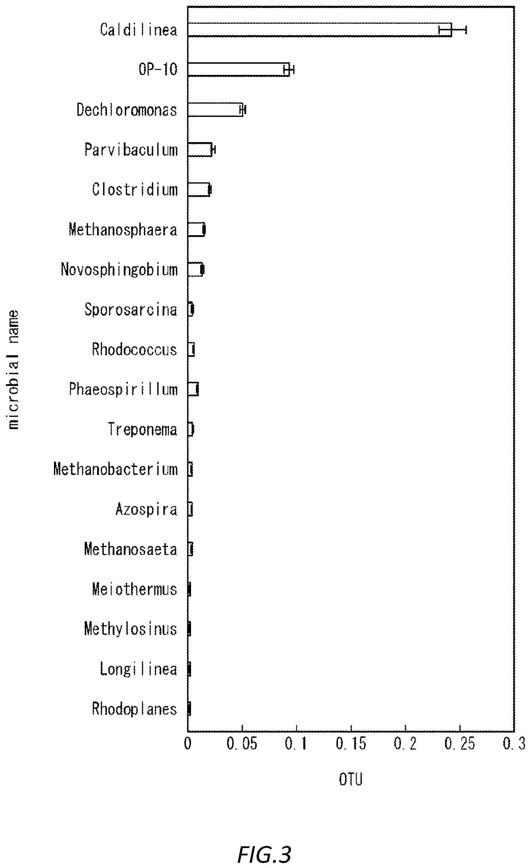

Metagenomic analysis was performed on the collected microbiota, types of microbial species and their proportions (OTU) were analyzed. FIG. 3 shows OTU values of microbial species having a high OTU value.

In addition, as a result of the metagenomic analysis, it was revealed that bacteria of genus Caldilinea, Candidate division OP10, genus Dechloromonas, genus Parvibaculum, genus Clostridium, genus Methanosphaera, genus Novosphingobium, genus Sporosarcina, genus Rhodococcus, genus Phaeospirillum, genus Treponema, genus Methanobacterium, genus Azospira, genus Methanosaeta, genus Meiothermus, genus Methylosinus, genus Longilinea, and genus Rhodoplanes were included in the microbiota.

### (Experimental example 2)

### (Main culture of microbiota)

First, waste gypsum was separated into gypsum and cardboard. The gypsum was crushed in a mortar, and the cardboard was cut to 10x1 mm. In addition, industrial glue (manufactured by Yayoi Chemical Industry Corporation), calcium sulfate dihydrate (Hayashi Pure Chemical Industry first class reagent), and the above described activated sludge were prepared.

The above described materials were added to each screw-capped glass test tube (1 to 7).
Test tube 1 Crushed gypsum (1.0 g)
Test tube 2 Industrial glue (1.0 g)
Test tube 3 Calcium sulfate dihydrate (0.5 g), and industrial glue (0.5 g)
Test tube 4 Crushed gypsum (0.5 g), and cut cardboard (0.5 g)
Test tube 5 Calcium sulfate dihydrate (1.0 g)
Test tube 6 Cut cardboard (1.0 g)
Test tube 7 None
Then, the test tubes (1 to 7) were sterilized in an autoclave at 121 degrees Celsius for 20 minutes.

Then, 10 mL of liquid including collected microbiota (activated sludge) was dispensed in the respective test tubes. Each test tube was placed in an incubator, and a main culture was performed at 30 degrees Celsius for 24 hours.

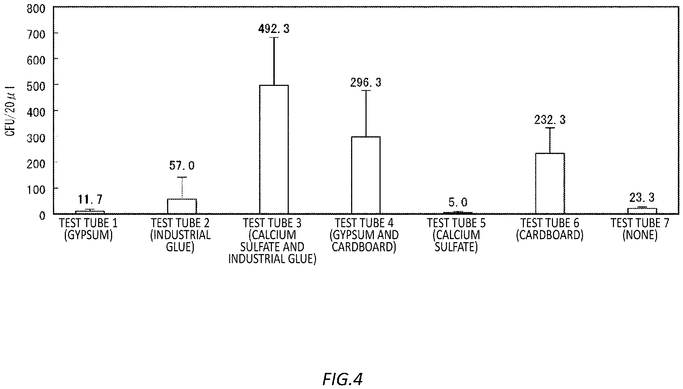

20 µL of culture solution after the main culture of test tubes 1 to 7 were each inoculated on normal broth culture medium with a cell spreader, and bacteria count tests were performed. FIG. 4 shows the results.

When cultured in test tube 4 (crushed gypsum and cut cardboard), proliferation of microorganisms was sufficient. This result shows that microorganisms can sufficiently proliferate when cultured in a culture medium including gypsum and cellulose. That is, it was revealed that microorganisms can sufficiently proliferate when a culture medium including waste gypsum including gypsum and cardboard is used.

When cultured in test tube 1 (crushed gypsum), test tube 5 (calcium sulfate dihydrate), proliferation of microorganisms was not sufficient. In addition, when cultured in test tube 2 (industrial glue), proliferation of microorganisms was not sufficient.

Test tube 1 and test tube 5 include calcium sulfate dihydrate, and test tube 2 includes starch. This shows that microorganisms cannot sufficiently proliferate in a culture medium including only one of calcium sulfate dihydrate or starch.

Meanwhile, in test tube 3 (calcium sulfate and industrial glue), proliferation of microorganisms was sufficient. This result shows that microorganisms can sufficiently proliferate in a culture medium including calcium sulfate and starch.

When cultured in test tube 6 (cut cardboard), proliferation of microorganisms was sufficient. This result shows that microorganisms can sufficiently proliferate when cultured in a culture medium including plant fiber derived from cardboard.

(Experimental example 3)

### (Decomposition of ammonia)

A test of ammonia decomposition was performed using the microbial preparation.

1 mL of ammonia water (Japanese Pharmacopoeia, NH₃ 9.5 to 10.5 w/v%) was dropped in a glass test tube of ϕ15 mm × 180 mm, and the opening was occluded with plastic wrap. 10 seconds after occlusion, the plastic wrap was pierced with a gas detection tube (for ammonia/manufactured by Gastec Corporation), gas inside the test tube was sucked, and this measurement value was considered an initial value.

In this gas detection tube, a reagent inside the detection tube reacts with ammonia, and changes color from white to yellow. Using this detection tube, ammonia concentration can be measured with consideration of number of suctions and color change.

After measuring the initial value, the test tube was covered with new plastic wrap, the plastic wrap was removed after 10 seconds, and deodorant was immediately sprayed inside the test tube. The opening of the test tube was immediately occluded with plastic wrap, the plastic wrap was pierced with the gas detection tube after 10 seconds, gas inside the test tube was sucked, and this measurement value was considered a value after processing.

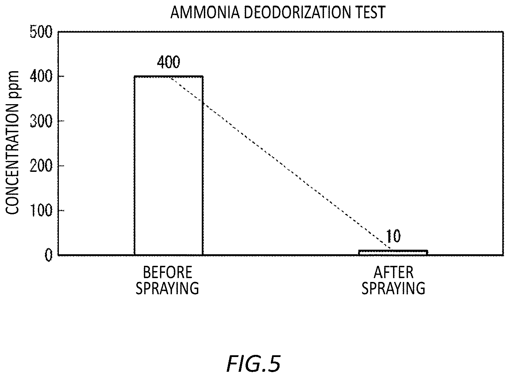

FIG. 5 shows the measurement results.

These results show that ammonia was decomposed by the microbial preparation.

### (Experimental example 4)

### (Deodorization test of a restroom)

A deodorization test of a restroom was performed using the microbial preparation.

The microbial preparation was sprayed on and input in two urinals in a public restroom (FRP manufactured urinal approximately 800 mmw x 1200 mmh). Change in ammonia concentration and change in odor were tested.

The ammonia concentration was measured using a gas detection tube (manufactured by Gastec Corporation 0 to 30 ppm). The odor was determined by olfaction of an evaluator.

### February 22, 2017

1.5 L of the microbial preparation was sprayed on the entire urinal, and ammonia concentration before and after spraying was measured using a gas detection tube (manufactured by Gastec Corporation 0 to 30 ppm). As a result, the ammonia concentration before spraying was 5 ppm, and the ammonia concentration after spraying was 0 ppm.

### February 24, 2017

Odor inside the restroom was confirmed again. 1 L of the microbial preparation was input in each urinal.

### March 11, 2017

It was confirmed that the odor inside the restroom was reduced by input of the microbial preparation.

2 L of the microbial preparation was sprayed on a dirty dripping part, surrounding area, and drain of the urinals, and 20 L of the microbial preparation was input in a urine reservoir.

As a result, the ammonia concentration before spraying was 15 ppm, and the ammonia concentration after spraying was 0 ppm.

### March 16, 2017

It was confirmed that the odor inside the restroom was maintained at a reduced level due to spraying and input of the microbial preparation on March 11.

### March 18, 2017

2 L of the microbial preparation was sprayed on the dirty dripping part, surrounding area, and drain of the urinals, and 20 L of the microbial preparation was input in the urine reservoir. As a result, the ammonia concentration before spraying was 2 ppm, and the ammonia concentration after spraying was 0 ppm.

### March 25, 2017

Due to spraying and input of the microbial preparation on March 18, the odor inside the entire restroom was reduced. However, the ammonia concentration in the urine reservoir was high.

1 L of the microbial preparation was sprayed on the dirty dripping part, surrounding area, and drain of the urinals, and 20 L of the microbial preparation was input in the urine reservoir. As a result, the ammonia concentration before spraying was 24 ppm, and the ammonia concentration after spraying was 0 ppm. In addition, as a result of a test by olfaction, it was confirmed that the odor inside the restroom was reduced by this time of spraying and input.

### March 27, 2017

It was confirmed that the odor inside the restroom was reduced to an extent that it was almost imperceptible by spraying and input performed on March 25, 2017.

These results show that odor causing substances such as ammonia were decomposed by the microbial preparation.

### (Experimental example 5)

### (Inputting the microbial preparation in a sludge digestion tank)

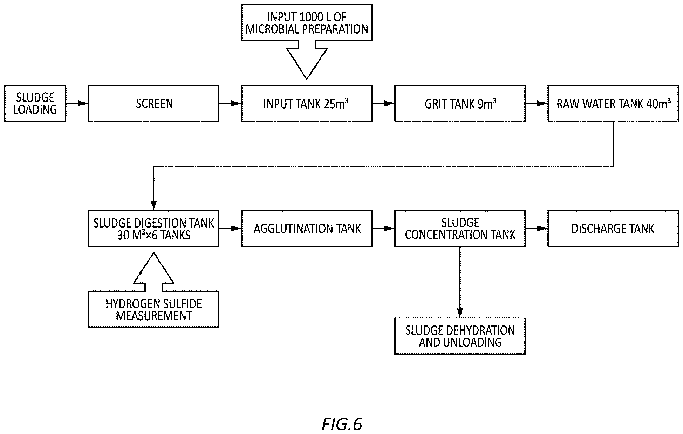

On August 1, 2014, 1000 L of the microbial preparation was input in a sludge digestion tank. Concentration of hydrogen sulfide gas was continuously measured in a gas phase part of the sludge digestion tank. FIG. 6 shows a flow of sludge treatment.

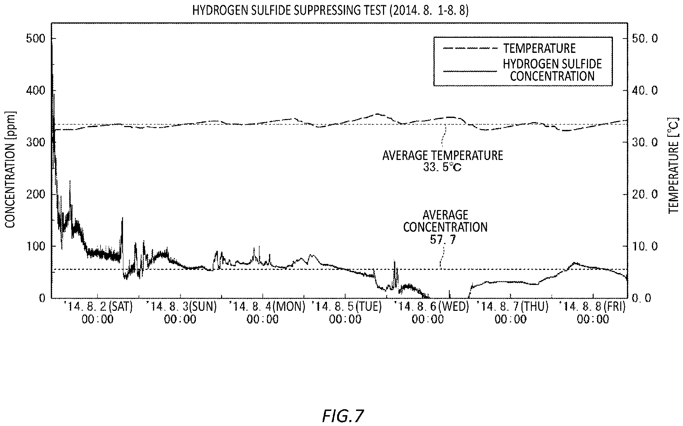

FIG. 7 shows measurement results of the hydrogen sulfide concentration. Hydrogen sulfide concentration before input was 400 to 500 ppm, and hydrogen sulfide concentration after input was approximately 50 ppm. This result shows that generation of hydrogen sulfide was reduced in the sludge treatment process by input of the microbial preparation.

### (Experimental example 6)

### (Enzyme activity of the microbial preparation)

Waste gypsum, calcium sulfate dihydrate (Hayashi Pure Chemical Industry first class reagent), and the above described activated sludge were prepared.

| | |
|---|---|
| Test tube A1 | None |
| Test tube A2 | Calcium sulfate dihydrate (0.5 g), and industrial glue (0.5 g) |
| Test tube A3 | Crushed waste gypsum |

Here, "crushed waste gypsum" includes gypsum and cardboard.

Then, test tubes 1A to 3A were sterilized in an autoclave at 121 degrees Celsius for 20 minutes.

Then, 10 mL of activated sludge was dispensed in the respective test tubes. Each test tube was placed in an incubator, and a main culture was performed at 30 degrees Celsius for 24 hours. Supernatant of each test tube were considered test examples A1 to A3.

For each culture solution, enzyme activity of exocellulase, endocellulase, and hemicellulase was measured by quantifying the amount of reducing sugars generated from cellulose or hemicellulose. A specific measurement method is shown below.

### Measurement method of enzyme activity:

300 mg each of microcrystalline cellulose (Avicel, manufactured by Sigma-Aldrich Corporation) carboxymethyl cellulose, and xylan were suspended in 30 mL of pure water to obtain a substrate suspension.

Then, 500 µL of each of the test examples A1 to A3 and 500 µL of each substrate suspension were mixed to obtain 1 mL of each mixed liquid. Then, each of these mixed liquids was reacted for three hours at 40 to 50 degrees Celsius to obtain a reacted solution. In addition, each of these mixed liquids was cooled with ice without being reacted to obtain an unreacted solution.

Then, concentration of reducing sugars such as glucose and cellobiose in the reacted solution and the unreacted solution was measured using dinitrosalicylic acid. A difference between the measurement value of the reacted solution and the measurement value of the unreacted solution was calculated and considered a reducing sugars generated amount in the test examples A1 to A3.

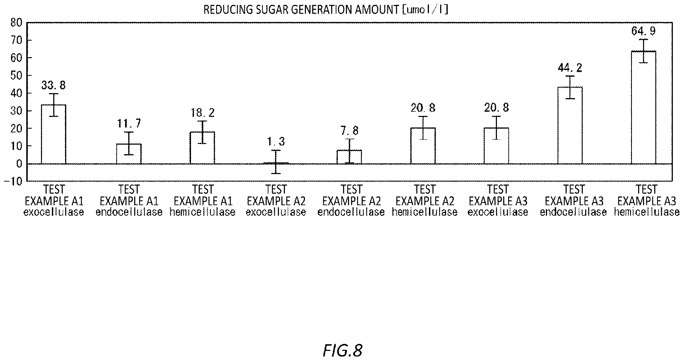

FIG. 8 shows calculated results of the reducing sugars generated amount. In FIG. 8, the vertical axis is µmol/L.

Test example A3 had higher enzyme activity of endocellulase in comparison to test example A1 and test example A2.

Test example A3 had significantly higher enzyme activity of hemicellulase in comparison to test example A1 and test example A2.

It was confirmed that by adding waste gypsum to microbiota and culturing it, enzyme activity of endocellulase and hemicellulase in the microorganisms was increased.

While carbohydrates, proteins and the like are classified as easily decomposable organic matter, cellulose and hemicellulose are classified as refractory organic matter.

Industrial glue includes starch, and is classified as easily decomposable organic matter. On the other hand, waste gypsum includes refractory organic matter since it includes cardboard.

It was confirmed from these results that by adding waste gypsum to microbiota, enzyme activity in microorganisms that decomposes refractory organic matter is increased.

Although the reason for such an effect is not clear, it is presumed as follows.

Many types of microorganisms exist in activated sludge. By adding waste gypsum to activated sludge and culturing it, proportion of microbial species that can decompose refractory organic matter and proliferate increases. In addition, expression level and activity of degrading enzymes for refractory organic matter in the microorganisms increases. It is presumed that as a result, the culture solution that has been cultured with waste gypsum added to activated sludge has expression levels and activity of degrading enzymes for refractory organic matter that are higher than a culture solution that has been cultured with calcium sulfate and industrial glue added to activated sludge.

### (Experimental example 7)

### (Cultivation test 1: microbial preparation)

The above described activated sludge, crushed waste gypsum, calcium sulfate dihydrate (Hayashi Pure Chemical Industry first class reagent), industrial glue (manufactured by Yayoi Chemical Industry Corporation), Kanuma black soil (manufactured by Marukei Corporation), and a Neubauer pot were prepared.

Here, "crushed waste gypsum" includes gypsum and cardboard.

The following liquid fertilizer raw materials 1 to 3 were prepared.
Liquid fertilizer raw material 1: solution of 10 parts by mass activated sludge added with 0.5 parts by mass crushed gypsum, and 0.5 parts by mass cut cardboard
Liquid fertilizer raw material 2: solution of 10 parts by mass pure water added with 0.5 parts by mass calcium sulfate dihydrate, and 0.5 parts by mass industrial glue
Liquid fertilizer raw material 3: 10 parts by mass activated sludge
Each liquid fertilizer raw material was placed in an incubator, cultured at 30 degrees Celsius for 24 hours to obtain undiluted liquid fertilizers 1 to 3.

Supernatant of the undiluted liquid fertilizers 1 and 3 were diluted 500 times with water, and liquid fertilizers of standard concentration 1 and 3 were prepared. The undiluted liquid fertilizer 2 was diluted 500 times with water, and a liquid fertilizer of standard concentration 2 was prepared. In addition, a solution of double concentration and a solution of triple concentration were prepared for each liquid fertilizer of standard concentration.

Total nitrogen amount, total phosphorus amount, total potassium amount, and C/N ratio were measured for the liquid fertilizers of standard concentration 1 to 3. The total nitrogen amount was measured using HPLC. Phosphorus and potassium were measured using an energy dispersive X-ray fluorescence spectrometer (Hitachi High-Tech Science, AMETEC SPECTRO XEPOS ED-XRF). Total carbon amount was measured by ignition loss method.

Table 1 shows the measurement results. In the table, values for moisture, total nitrogen (N), total phosphorus (P), and total potassium (K) refers to % by mass in relation to total amount of each liquid fertilizer (100%).

**(Table 1)**

| | MOISTURE | N | P | K | C/N RATIO |
|---|---|---|---|---|---|
| LIQUID FERTILIZER 1 (STANDARD CONCENTRATION) | 89.9 | 0.005 | 0.002 | 0.032 | 2 |
| LIQUID FERTILIZER 2 (STANDARD CONCENTRATION) | 90.0 | 0.005 | 0.002 | 0.032 | 2 |
| LIQUID FERTILIZER 3 (STANDARD CONCENTRATION) | 99.8 | 0.005 | 0.002 | 0.032 | 2 |

Soil used in each test example was prepared by the following procedure.

After passing Kanuma black soil in a sieve (2 mm mesh), it was air dried. 500 mL of the air dried soil was put in a Neubauer pot.

Moisture content in the soil in the pot was adjusted to 50 to 60% of maximum water capacity.

Here, maximum water capacity is defined as follows. A grooved funnel with a diameter of approximately 110 mm is placed on a 100 mL graduated cylinder. Then, three types or two types of filter paper with a diameter of approximately 185 mm that have been moistened with water are placed in this funnel. Then, 100 g of soil that is air dried soil dried at 100 degrees Celsius for five hours is put in that filter paper, and 100 mL of water is gently poured from a soil surface. The maximum water capacity refers to amount of water held in soil determined from amount of water dripped after confirming that filtrate has finished dripping.

20 seeds of "Early harvest large leaf Komatsuna Misugi (manufactured by Sakata Seed Corporation)" were sown in a grid shape in each test example so that each of the seeds was equally spaced. After sowing, air dried soil was placed on top of the seeds to an extent that the seeds were hidden.

In each test example, as shown in the following, the liquid fertilizers 1 to 3 or water was supplied. In each test example, cultivation was performed using two pots.

The liquid fertilizers 1 to 3 and the control water was supplied to the soil in the pots three times one time a week after the seeds germinated. Amount supplied per one time was 21 mL.
No liquid fertilizer:

| | |
|---|---|
| Test example B0 | Water |

Standard amount:

| | |
|---|---|
| Test example B11 | Liquid fertilizer 1 (standard concentration) |
| Test example B21 | Liquid fertilizer 2 (standard concentration) |
| Test example B31 | Liquid fertilizer 3 (standard concentration) |

Double amount:

| | |
|---|---|
| Test example B12 | Liquid fertilizer 1 (double concentration) |
| Test example B22 | Liquid fertilizer 2 (double concentration) |
| Test example B32 | Liquid fertilizer 3 (double concentration) |

Triple amount:

| | |
|---|---|
| Test example B13 | Liquid fertilizer 1 (triple concentration) |
| Test example B23 | Liquid fertilizer 2 (triple concentration) |
| Test example B33 | Liquid fertilizer 3 (triple concentration) |

Total nitrogen amount and amount of phosphorus and potassium were measured for total amount (that is, 61 mL) of each liquid fertilizer added to the pots. Table 2 shows the results. Units are mg/pot.

**(Table 2)**

| | N | P | K |
|---|---|---|---|
| LIQUID FERTILIZER 1 (STANDARD CONCENTRATION) | 0.3 | 12.6 | 19.9 |
| LIQUID FERTILIZER 1 (DOUBLE CONCENTRATION) | 0.2 | 12.2 | 19.5 |
| LIQUID FERTILIZER 1 (TRIPLE CONCENTRATION) | 0.1 | 12.6 | 20.2 |
| LIQUID FERTILIZER 2 (STANDARD CONCENTRATION) | 0,3 | 12.0 | 19.6 |
| LIQUID FERTILIZER 2 (DOUBLE CONCENTRATION) | 0.3 | 11.5 | 19.4 |
| LIQUID FERTILIZER 2 (TRIPLE CONCENTRATION) | 0.1 | 11.5 | 19.0 |
| LIQUID FERTILIZER 3 (STANDARD CONCENTRATION) | 0.4 | 12.0 | 19.8 |
| LIQUID FERTILIZER 3 (DOUBLE CONCENTRATION) | 0.4 | 12.8 | 20.3 |
| LIQUID FERTILIZER 3 (TRIPLE CONCENTRATION) | 0.1 | 12.6 | 20.5 |
| WATER | 0.7 | 12.6 | 20.5 |

10 days after cultivation started, moisture content in the soil was adjusted to 50 to 60% of maximum water capacity. From 10 days and after cultivation started, water was supplied appropriately in accordance with crop growth.

Cultivation temperature was maintained at 15 to 25 degrees Celsius.

Germination rate of the seeds and leaf length were measured by a method shown below. The results were shown in Tables 3 to 5.

### Germination rate:

After 5 days from the day the seeds were sown, a proportion of the 20 sown seeds that germinated was calculated as %.

### Leaf length:

After three weeks from the day the seeds were sown, the leaf length was measured. Here, the "leaf length" refers to a linear distance from a base of a leaf to a tip of the leaf (that is, length of a leaf blade). In addition, in Tables 3 to 5, the "leaf length" is described as a longest measurement value of leaf length in each pot.

**(Table 3)**

| STANDARD CONCENTRATION | GERMINATION RATE (%) | LEAF LENGTH (cm) |
|---|---|---|
| TEST EXAMPLE B11 POT 1 | 100 | 3.0 |
| TEST EXAMPLE B11 POT 2 | 100 | 4.0 |
| TEST EXAMPLE B21 POT 1 | 100 | 3.0 |
| TEST EXAMPLE B21 POT 2 | 100 | 3.0 |
| TEST EXAMPLE B31 POT 1 | 90 | 3.0 |
| TEST EXAMPLE B31 POT 2 | 100 | 2.5 |
| TEST EXAMPLE B0 POT 1 | 90 | 2.5 |
| TEST EXAMPLE 80 POT 2 | 100 | 3.0 |

**(Table 4)**

| DOUBLE CONCENTRATION | GERMINATION RATE (%) | LEAF LENGTH (cm) |
|---|---|---|
| TEST EXAMPLE B12 POT 1 | 100 | 3.5 |
| TEST EXAMPLE B12 POT 2 | 95 | 3.0 |
| TEST EXAMPLE B22 POT 1 | 80 | 3.0 |
| TEST EXAMPLE B22 POT 2 | 100 | 3.0 |
| TEST EXAMPLE B32 POT 1 | 100 | 3.0 |
| TEST EXAMPLE B32 POT 2 | 90 | 3.0 |

**(Table 5)**

| TRIPLE CONCENTRATION | **GERMINATION** RATE (%) | LEAF LENGTH (cm) |
|---|---|---|
| TEST EXAMPLE B13 POT 1 | 100 | 3.0 |
| TEST EXAMPLE B13 POT 2 | 85 | 3.0 |
| TEST EXAMPLE B23 POT 1 | 85 | 3.0 |
| TEST EXAMPLE B23 POT 2 | 95 | 3.0 |
| TEST EXAMPLE B33 POT 1 | 100 | 3.0 |
| TEST EXAMPLE B33 POT 2 | 100 | 2.8 |

### Fresh weight:

21 days after the day the seeds were sown, the cultivated plants were collected, total weight was measured, and this was considered fresh weight. Unit of the fresh weight is total weight of the plant collected in one pot (g). Table 9 shows the results.

In the test example using each liquid fertilizer, the germination rates were normal.

At standard concentration and double concentration, the leaf length in the test example using liquid fertilizer 1 (culture of activated sludge and waste gypsum) was longer than the leaf length of liquid fertilizer 2 (mixture of water, calcium sulfate, and industrial glue) and liquid fertilizer 3 (activated sludge).

Typically, in soil, easily decomposable organic matter is in a state of decomposition by microorganisms existing in the soil. By adding a microbial preparation produced by culturing microbiota with waste gypsum to soil, refractory organic matter in the soil is decomposed.

This microbial preparation includes a calcium component and a sulfur component supplied from waste gypsum. In addition, calcium sulfate included in waste gypsum shows neutrality.

It is presumed by this synergistic effect, crop growth is improved in a soil to which the microbial preparation is added.

### (Experimental example 8)

### (Cultivation test 2: compost)

Cow manure, the above described activated sludge, a microbial preparation, Kanuma black soil (manufactured by Marukei Corporation), and a Neubauer pot were prepared.

Cow manure from Shizuoka prefecture was used.

The microbial preparation used was one in which 0.5 parts by mass of crushed gypsum and 0.5 parts by mass of cut cardboard were added to 10 parts by mass of activated sludge and cultured at 30 degrees Celsius for 24 hours.

The following compost raw materials 1 to 3 were prepared.

Compost raw material 1: cow manure (approximately 120 g) and pure water (approximately 20 g) were mixed to obtain a mixture.

Compost raw material 2: cow manure (approximately 120 g) and supernatant of activated sludge (approximately 20 g) were mixed to obtain a mixture.

Compost raw material 3: cow manure (approximately 120 g) and supernatant of microbial preparation (approximately 20 g) were mixed to obtain a mixture.

In each compost raw material, moisture content was 40 to 60%. The compost raw materials 1 to 3 were fermented at 40 degrees Celsius for four weeks to obtain composts 1 to 3. During fermentation, the mixtures were sufficiently kneaded once a week. This is equivalent to a cutback process in a general compost production method. After fermentation, generation of transparent or white mycelium, generally seen upon completion of compost, was confirmed.

Similarly to experimental example 7, total nitrogen amount, total phosphorus amount, total potassium amount, and C/N ratio were measured for the composts 1 to 3.

Table 6 shows the measurement results. In the table, values for moisture, total nitrogen, total phosphorus, and total potassium refers to % by mass in relation to total amount of each compost (100%).

**(Table 6)**

| | MOISTURE | TOTAL NITROGEN | TOTAL PHOSPHORUS | TOTAL CALCIUM | C/N RATIO |
|---|---|---|---|---|---|
| COMPOST 1 | 61 | 1.12 | 0.66 | 1.62 | 16.3 |
| COMPOST 2 | 61 | 0.50 | 1.41 | 2.74 | 36.5 |
| COMPOST 3 | 61 | 1.00 | 1.05 | 2.39 | 18.2 |

In each test example, 500 mL of air dried soil made by the same method as in experimental example 7 was prepared, and moisture content of the soil was adjusted to 50 to 60% of maximum water capacity.

Then, a cultivation soil was obtained by mixing the soil above and the following amounts of each compost. This cultivation soil was put in a Neubauer pot. In each test example, cultivation was performed using two pots.
No compost:

| | |
|---|---|
| Test example C0 | No compost |

Standard amount:

| | |
|---|---|
| Test example C11 | Compost 1 6 g |
| Test example C21 | Compost 2 6 g |
| Test example C31 | Compost 3 6 g |

Double amount:

| | |
|---|---|
| Test example C12 | Compost 1 12 g |
| Test example C22 | Compost 2 12 g |
| Test example C32 | Compost 3 12 g |

Triple amount

| | |
|---|---|
| Test example C13 | Compost 1 18 g |
| Test example C23 | Compost 2 18 g |
| Test example C33 | Compost 3 18 g |

Total nitrogen amount and amount of phosphorus and potassium were measured for each compost added to the pots.

Table 7 shows the results. Unit is mg/pot.

**(Table 7)**

| | N | P | K |
|---|---|---|---|
| COMPOST 1 6g | 8.3 | 2.8 | 0.4 |
| COMPOST 1 12g | 17.2 | 2.8 | 0.6 |
| COMPOST 1 18g | 22.3 | 8.3 | 0.9 |
| COMPOST 2 6g | 5.4 | 2.8 | 0.4 |
| COMPOST 2 12g | 11.5 | 5.6 | 0.7 |
| COMPOST 2 18g | 13.8 | 8.2 | 1.1 |
| COMPOST 3 6g | 7.7 | 2.8 | 0.4 |
| COMPOST 3 12g | 16.1 | 5.6 | 0.7 |
| COMPOST 3 18g | 20.6 | 8.4 | 1.1 |

20 seeds of "Early harvest large leaf Komatsuna Misugi (manufactured by Sakata Seed Corporation)" were sown in a grid shape in each test example so that each of the seeds was equally spaced. After sowing, air dried soil was placed on top of the seeds to an extent that the seeds were hidden.

Similarly to experimental example 7, moisture content of cultivation soil in the pots were adjusted appropriately by irrigation to 50 to 60% of maximum water capacity.

Cultivation was performed in a plastic greenhouse, and temperature inside the greenhouse was adjusted to be 25 degrees Celsius on average. When room temperature inside the plastic greenhouse became 15 degrees Celsius or less, it was heated.

Irrigation was done once every two days in principle. Irrigation amount was adjusted according to crop growth.

### Germination rate:

4 days after and 7 days after the day the seeds were sown, germination rate was calculated.

The germination rate is a proportion of germinated seeds (%) in relation to the total 20 sown seeds (100%). Table 8 shows the results.

**(Table 8)**

| | GERMINATION RATE (%) | |
|---|---|---|
| | 4 DAYS AFTER SOWING | 7 DAYS AFTER SOWING |
| TEST EXAMPLE C11 | 50 | 50 |
| TEST EXAMPLE C21 | 45 | 85 |
| TEST EXAMPLE C31 | 65 | 70 |
| TEST EXAMPLE C12 | 30 | 45 |
| TEST EXAMPLE C22 | 55 | 70 |
| TEST EXAMPLE C32 | 50 | 75 |
| TEST EXAMPLE C13 | 20 | 40 |
| TEST EXAMPLE C23 | 40 | 45 |
| TEST EXAMPLE C33 | 50 | 55 |

### Fresh weight:

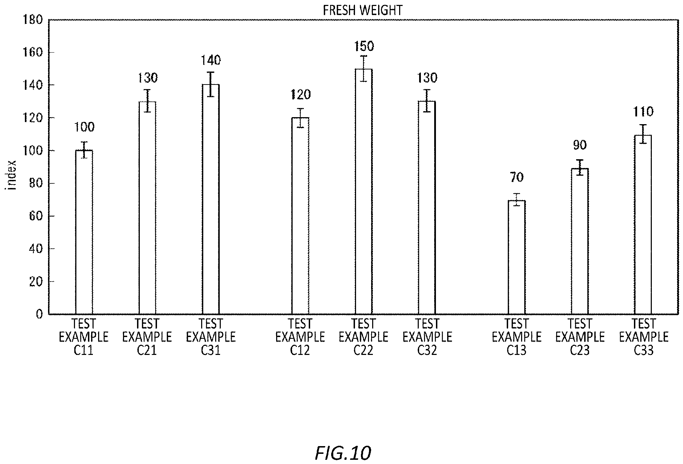

45 days after the day the seeds were sown, the cultivated plants were collected, total weight was measured, and this was considered fresh weight. FIG. 10 shows the results.

In FIG. 10, the fresh weight is shown as a relative value with test example C11 set as 100.

Measurement of total nitrogen amount in soil after cultivation:
After collecting all plants above and in the soil, total nitrogen amount included in the cultivation soil after cultivation was measured using a digital pack test (absorbance method, Kyoritsu Chemical-Check Lab Corporation).

Measurement of nitrate nitrogen amount in plant:
Nitrate nitrogen amount included in the collected plants was measured using a compact nitrate ion meter (manufactured by HORIBA Corporation, LAQUAtwin).

In addition, measurements were made for the soil of test example C0 in which compost was not mixed.

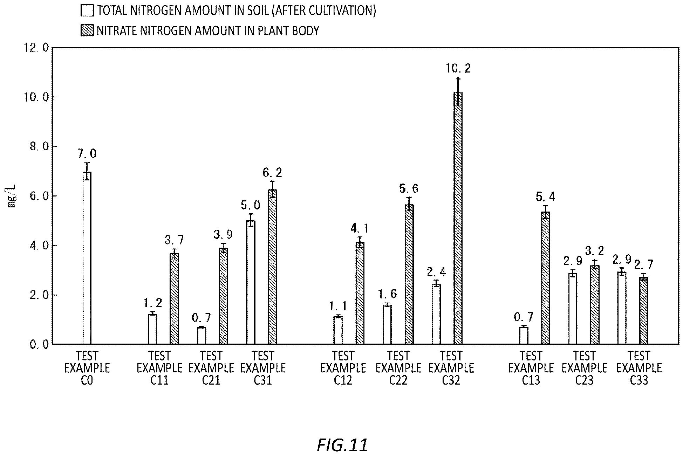

FIG. 11 shows the measurement results.

Measurement of electrical conductivity of soil extract after cultivation:
50 mL of water was added to 10 g of soil after cultivation and shaken to obtain a mixture. Then, the mixture was filtered with filter paper to obtain a filtrate. Electrical conductivity of this filtrate was measured using an electrical conductivity meter.

FIG. 11 shows the measurement results.

Measurement of pH of soil extract after cultivation:
pH was measured using a pH meter for the filtrate prepared in the measurement of electrical conductivity.

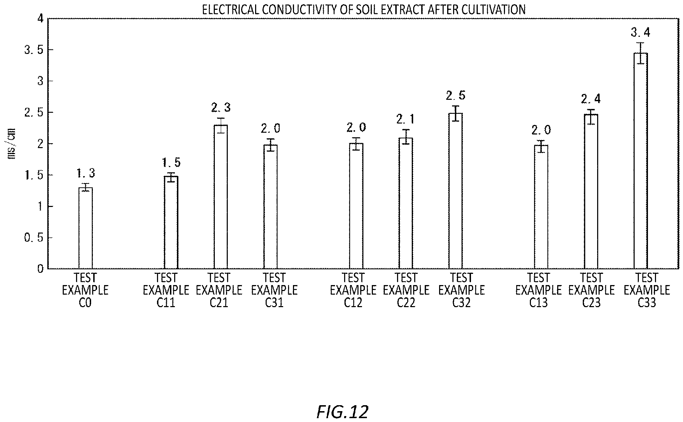

FIG. 12 shows the measurement results.

Germination rate 7 days after sowing in test examples C11, C12, and C13 in which compost 1 (fermented product of cow manure and water) was added were each 50% or less.

Germination rate 7 days after sowing in test examples C21 and C22 in which compost 2 (fermented product of cow manure and activated sludge) was added were each 70% or more.

Germination rate 7 days after sowing in test examples C31 and C32, in which compost 3 (fermented product of cow manure and microbial preparation) was added were each 70% or more.

It is presumed that compost 1 caused germination problems to occur since ripeness of the cow manure was not sufficient. It is presumed that compost 2 and compost 3 increased germination rate since ripeness of the cow manure was more increased than in compost 1.

Among the test examples C11, C21, and C31 to which a standard amount of the composts 1 to 3 were added, for the fresh weight, test example C31 was heavier than test example C21, and test example C21 was heavier than test example C11.

It is presumed that by adding activated sludge to cow manure and fermenting it, content of nutrients for plants was increased and plant growth was enhanced more in test example C21, in comparison to test example C11.

It is presumed that by adding the microbial preparation (that is, activated sludge, gypsum, and cardboard) to cow manure and fermenting it, content of nutrients for plants was further increased and plant growth was further enhanced in test example C31, in comparison to test example C21.

As shown in Table 6, before cultivation, the cultivation soil to which compost 2 or compost 3 was added had less total nitrogen amount than the cultivation soil to which compost 1 was added.

On the other hand, as shown in FIG. 11, when the double amount of compost was added, nitrate nitrogen amount in the plant bodies of test example C22 and test example C32 were higher than that in test example C12. That is, it was confirmed that when compost 2 or compost 3 was used, nitrogen fixation in the plant body was more increased than with compost 1.

In addition, when the double amount of compost was used, total nitrogen amount in the soil after cultivation in test example C22 and test example C32 was higher than that in test example C12. That is, it is presumed that by using compost 2 or compost 3, nitrogen compounds in the soil are suppressed from volatizing or being dissolved in and flowing out of water, and nitrogen compounds is more easily held in the cultivation soil.

In the cultivation soil to which compost 1 was added, the higher the added amount of compost, the more the electrical conductivity and pH decreased. This is presumed to be due to nitrate nitrogen derived from compost 1 being added to the cultivation soil. In the cultivation soil to which compost 2 or compost 3 was added, pH was high than in the soil to which compost 1 was added. That is, it was confirmed that pH does not decrease even when the added amount of compost 2 or compost 3 is increased

Compost is made by decomposing and mineralizing easily decomposable organic matter included in residue derived from animals and plants. Here, as described above in experimental example 6, by adding waste gypsum to microbiota and culturing it, enzyme activity in microorganisms that decompose refractory organic matter is increased.

That is, by adding a microbial preparation including microbiota and waste gypsum to compost raw material such as cow manure and fermenting it, decomposition of refractory organic matter in the compost raw material is enhanced. It is presumed that in this way, effect of compost is increased, and crop growth is improved.

Soil may become alkaline and cause problems with crop growth to occur by adding lime-based fertilizer to the soil. Meanwhile, compost including waste gypsum is neutral. In addition, by using waste gypsum, atoms of calcium for strengthening cell walls of a plant and sulfur for raw material of amino acids can be supplied to the soil.

That is, compost of the present invention can improve crop growth by using a combination of microbiota and waste gypsum.

### INDUSTRIAL APPLICABILITY

According to the present invention, waste gypsum can be effectively utilized, and a technique for producing a microbial preparation having organic matter decomposition ability and a technique for producing compost that can improve yield of agricultural crops can be provided.

## Claims

1. A production method of a microbial preparation, comprising:
mixing crushed waste gypsum with a culture medium including microorganisms to obtain a mixed liquid; and
culturing the mixed liquid, wherein
particle diameter of the crushed waste gypsum is 30 mm or less.

2. The production method according to claim 1, wherein
the culturing the mixed liquid is performed under aerobic conditions.

3. The production method according to claim 1, wherein
the culturing the mixed liquid is performed at a temperature of 10 to 50 degrees Celsius.

4. The production method according to claim 1, wherein
the culture medium includes one type or more selected from a group formed of starch and plant fiber, and calcium sulfate.

5. The production method according to claim 1, further comprising
determining that arsenic and cadmium content in the waste gypsum is a certain value or less.

6. The production method according to claim 1, wherein
content of the waste gypsum in relation to total mass of the mixed liquid is 0.01 to 30% by mass.

7. The production method according to any one of claims 1 to 6, wherein
the microorganisms are microorganisms collected from activated sludge.

8. A microbial preparation, comprising:
microorganisms, crushed waste gypsum, and water, wherein
particle diameter of the crushed waste gypsum is 30 mm or less.

9. A compost production method, comprising adding microorganisms and waste gypsum to organic matter and fermenting it.

10. A composting material, comprising microorganisms, waste gypsum, and organic matter.

11. A compost fermentation product, made by fermenting microorganisms, waste gypsum, and organic matter.

12. A culture soil, comprising the compost fermentation product according to claim 11.
